# EUROPEAN PATENT SPECIFICATION

(11) **EP 2 049 223 B1**
(45) Date of publication and mention of the grant of the patent: **03.01.2018**
(21) Application number: 07797059.8
(22) Date of filing: 31.07.2007
(51) Int. Cl.: B01D 35/00, B01D 24/32, B01D 33/067, B01L 3/02, B04B 5/04, A61M 1/36

(54) **APPARATUS AND METHOD FOR PREPARING PLATELET RICH PLASMA AND CONCENTRATES THEREOF**
VORRICHTUNG UND VERFAHREN ZUR HERSTELLUNG VON PLÄTTCHENREICHEM PLASMA UND KONZENTRATEN DAVON
APPAREIL ET PROCÉDÉ DE PRÉPARATION D'UN PLASMA RICHE EN PLAQUETTES ET CONCENTRÉS DE CE PLASMA

(30) Priority: 31.07.2006 US 834550 P
(43) Date of publication of application: 22.04.2009
(73) Proprietor: Hanuman LLC, San Francisco, CA 94124 (US); Biomet Biologics, LLC, Warsaw, Indiana 46582 (US)
(72) Inventor: DORIAN, Randel, San Diego, California 92117 (US); LEACH, Michael D., Warsaw, Indiana 46582 (US)
(74) Representative: Handley, Matthew Edward
(86) International application number: PCT/US2007/017055
(87) International publication number: WO 2008/016574

(56) References cited:
- EP-A2- 0 272 915
- WO-A1-94/06535
- US-A- 4 921 473
- US-A- 5 549 834
- US-A- 5 885 239
- US-B2- 6 764 531

## Description

### FIELD

This disclosure relates to a device and method for preparing platelet-plasma concentrates with wound healing properties for use as a tissue sealant, adhesive, etc. The concentrates have a fully active (un-denatured) fibrinogen concentration that is greater than the concentration of fibrinogen in whole blood and a platelet concentration that is greater than the concentration of platelets in whole blood.

### BACKGROUND

Blood can be fractionated, and the different fractions of the blood are useful for different medical needs. Under the influence of gravity or centrifugal force, blood can separate into three layers. At equilibrium, the top low-density layer is a straw-colored clear fluid called plasma. Plasma is a water solution of salts, metabolites, peptides, and many proteins ranging from small (insulin) to larger molecules (complement components).

The bottom, high-density layer is a deep red viscous fluid comprising unnucleated red blood cells (erythrocytes) specialized for oxygen transport. The red color is imparted by a high concentration of chelated iron or heme that is responsible for the erythrocytes' high specific gravity. The relative volume of whole blood that consists of erythrocytes is called the hematocrit, and in normal human beings this can range from about 30% to about 60%, such as about 37% to about 52% of whole blood.

The intermediate layer can be the smallest, appearing as a thin white band above the erythrocyte layer and below the plasma layer; this is called the buffy coat. The buffy coat itself has two major components, nucleated leukocytes (white blood cells) and anuclear smaller bodies called platelets (or thrombocytes). Leukocytes confer immunity and contribute to debris scavenging. Platelets seal ruptures in blood vessels to stop bleeding, and deliver growth and wound healing factors to a wound site. Slower speed centrifugation or shorter duration centrifugation permits separation of erythrocytes and leukocytes from plasma, while the smaller platelets remain suspended in the plasma, resulting in platelet rich plasma (PRP).

U.S. Patent No. 5,585,007 identifies methods for making plasma concentrates from whole blood for use in wound healing and as a tissue sealant. This device, designed for placement in a medical laboratory or surgical amphitheatre, uses a disposable cartridge for preparing tissue sealant. The device was particularly applicable for stat preparations of autologous tissue sealants. Preparation in the operating room of about 5 ml of sealant from about 50 ml of patient blood required less than about 15 minutes. There was reduced risk of tracking error because preparation could take place in the operating room during the surgical procedure. Chemicals added could be limited to anticoagulant (e.g., citrate) and calcium chloride. The disposable cartridge could fit in the palm of the hand and was hermetically sealed to reduce exposure to patient blood and to ensure sterility. Adhesive and tensile strengths of the product were comparable or superior to pooled blood fibrin sealants made by precipitation methods. Use of antifibrinolytic agents (such as aprotinin) was not necessary because the tissue sealant contained high concentrations of natural inhibitors of fibrinolysis from the patient's blood.

This device used a new sterile disposable cartridge with the separation chambers for each run. Since the device was designed to be used in a normal medical setting with ample power, the permanent components were designed for long-term durability, safety and reliability, and were relatively heavy, using conventional centrifuge motors and accessories.

Small, self-contained centrifugal devices for obtaining platelet concentrates from blood are described in, copending U.S. Pat Application Serial No. 10/394,828 filed March 21, 2003,

This device separates blood into erythrocyte, plasma, and platelet layers. The device can be used to selectively remove the platelet layer as a platelet concentrate, that is, platelets suspended in a minimal amount of plasma.

Platelet rich plasma is a concentrated platelet product that can be produced from whole blood through commercially available systems, resulting in varying levels of platelet concentration. Platelets play a crucial role in the signaling cascade of normal wound healing. Activated platelets release the contents of their α-granules resulting in a deposition of powerful growth factors such as platelet derived growth factor (PDGF), transforming growth factor 'β-(TGF-β)', vascular endothelial growth factor (VEGF), and epidermal growth factor (EGF). PRP has been used in many different clinical applications, demonstrating the effectiveness and importance of the product for a variety of medical procedures. For example, percutaneous application of PRP to patients with severe lateral epicondylitis, 'i.e. tennis elbow' resulted in improved elbow function and reduced pain. Early maturation of bony fusion was observed when platelet concentrate was used during lumbar spinal fusions. Chronic diabetic foot ulcers treated with PRP achieved increased healing rates compared to the control group receiving standard care. Studies by S. Bhanot, and J. C Alex, FACIAL PLASTIC SURGERY, 18(1): 27-33 (2002) show decreased formation of hematoma and seroma, decreased postoperative swelling, and improved healing time for plastic surgeries that included PRP in the treatment. Further, during dental surgeries, the use of PRP has improved bone regeneration around implants.

PRP have demonstrated numerous clinical benefits to patients. Concentrations of at least about 1,000x10³ platelets/*µ*L may be useful. The system described in copending U.S. Pat. Application Serial No. 10/394,828 can provide platelets up to about 8 times baseline concentration, and the normal human platelet range is about 200x10³ platelets/*µ*L to about 400x10³ platelets/*µ*L. This means a highly effective concentrate in a range of about 1,600x10³ platelets/*µ*L to about 3,200×10³ platelets/*µ*L.

EP0272915 discloses a pipette tip which separates substances such as blood ceils and plasma by virtue of rotation. The pipette tip comprises a body wall having an axis of symmetry and shaped to define a liquid confining cavity. The wall is provided with a dispensing aperture at one end and an annular trap cavity at the other end, which holds the denser phase.

US5885293 discloses a rotor for centrifuging and separating blood. The rotor comprises a circumferential boundary wall and an inner core. An inflatable diaphragm is provided about the inner core and is inflated to reduce the volume of a processing chamber within the rotor. The diaphragm is also inflated to excel fluid from the processing chamber.

### SUMMARY

A PRP (Platelet Rich Plasma) separator assembly can comprise a cylindrical outer wall closed at the top by an upper plate and closed at the bottom. The outer wall has an inner surface. A cylindrical inner wall concentric with a cylindrical outer wall can having a top edge and a bottom. The inner wall defines a central axis. The bottom of the inner wall is closed by a sloped bottom plate having a central opening, the bottom plate has an upper surface sloped down to a central opening. The top edge of the inner wall terminates at a distance from the upper plate to define an annular erythrocyte passageway therebetween. The inner wall has an outer surface and an inner surface that slopes radially inward from a top edge to a bottom at an angle of from about 0.2 to about 5 degrees relative to a central axis of the inner wall. A cylindrical depth filter is positioned between the inner surface of the outer wall and the outer surface of the inner wall in communication with the inner wall through the erythrocyte passageway.

The distance between the top of the inner wall and the upper plate can be from about 0.02 to about 50 mm, such as about 0.02 mm to about 10 mm. The depth filter can extend beyond the top edge of the inner wall. According to various, embodiments, the upper plate has a bottom surface, and the depth filter extends to the bottom surface.

For producing platelet rich plasma concentrate for wound healing, the depth filter can have the capacity to accept up to or more than about 85 percent of the hematocrit value of a patent's blood and less than a major portion of the platelet rich plasma remaining after the erythrocytes separation from a patient's blood.

For producing platelet rich plasma concentrate for hemostasis, the depth filter can have the capacity to accept up to or more than about 97 percent of the hematocrit value of a patent's blood and less than a major portion of the platelet rich plasma remaining after the erythrocytes separation from a patient's blood.

For all applications, the depth filter can have the capacity to accept up to or more than about 99 percent of the hematocrit value of a patent's blood and less than a major portion of the platelet rich plasma remaining after the erythrocytes separation from a patient's blood.

The depth filter can have the capacity to accept about 100 percent of the hematocrit value of a patent's blood and less than a major portion of the platelet rich plasma remaining after the erythrocytes separation from a patient's blood.

The PRP separator can be mounted for rotation about an outlet tube concentric with the central axis.

The separation chamber can include a balanced array of separator plates extending radially inward from the inner wall and upward from the bottom plate, the separation chamber can be balanced for substantially vibration-free rotation about the central axis.

The PRP separator-concentrator assembly can comprise the PRP separation assembly described above in combination with a PRP concentrator assembly. The concentration assembly has a PRP concentration sump; an axially concentric rigid stationary outlet tube secured to the housing and extending through the PRP separation assembly to the PRP concentrate sump; and the PRP separation assembly is attached to and positioned above the PRP concentration assembly to form a combined separator-concentrator assemblage that is rotatable about the outlet tube.

A PRP separator-concentrator can include a PRP concentrator that comprises a concentration chamber having a floor for supporting desiccating beads and a wall with at least one opening covered or closed with a screen, the screen having openings that are sized to retain the desiccating beads in the concentration chamber, the concentration chamber being surrounded by an outer wall with a sloped floor secured thereto, the sloped floor including at its center, a PRP concentrate sump.

A PRP separator-concentrator can include a stationary bead rake that is secured to a stationary tube and extends outward therefrom, the rake having distal ends that are spaced at a distance from the upright screen supports. The concentrator chamber can contain sufficient desiccating beads to remove enough water to produce a product having from above one time up to about four times a base concentration or higher.

A process for separating platelet rich plasma from blood comprising a plasma, erythrocytes and platelets employs a device comprising cylindrical inner wall having a top edge and a central axis surrounded by a depth filter having a capacity to receive all of the erythrocytes in the blood but insufficient to receive all of the plasma in the blood, the inner surface of the inner wall having an angle of about 0.2 to about 20 degrees, such as about 0.2 degrees to about five degrees, from the central axis of the inner wall. The process can include: a) spinning the inner cylinder about its central axis at a speed that centrifugally separates the erythrocytes from the plasma and platelets and causes the erythrocytes to slide up the inner surface; b) continuing spinning for a time sufficient to allow the erythrocytes to flow up the inner surface and over the top edge into the depth filter, leaving platelet enriched plasma behind in the inner cylinder; c) slowing or discontinuing the spinning to permit the platelet enriched plasma to flow to the bottom of the inner cylinder.

The inner surface of the inner wall can be segmented by radially extending plates into separation zones, the plates maintaining substantially balanced distribution of the blood in the separation zones during rotation of the separation chamber, thereby reducing vibration and erythrocyte displacement from the depth filter. The rotational speed of the separation chamber can be accelerated to centrifugal speeds at a rate that allows balanced distribution of blood in the separation zones. After the centrifuging is complete, the rotation speed of the separation chamber can be decelerated to below centrifugal speeds at a rate that allows balanced distribution of the PRP in the separation zones. The acceleration and deceleration process can reduce vibration and erythrocyte displacement from the depth filter. The process can include separating platelet rich plasma from blood according to the above process and then concentrating the platelet rich plasma by contacting the platelet rich plasma with desiccating beads in a rotating concentrating chamber while the beads are stirred with a rake to form a platelet rich plasma concentrate.

When the concentrating chamber includes an outer screened cylinder confining the desiccating beads, the platelet rich plasma concentrate can be separated from the beads by rotating the concentrating chamber about its central axis at a speed that separates platelet rich plasma concentrate from the beads.

According to various embodiments, a process for preparing platelet rich plasma concentrate for wound healing, at least about 85 volume percent of the hematocrit value of the blood and less than a major portion of the erythrocyte depleted platelet rich plasma can remain after the erythrocyte fraction is retained by the depth filter.

According to various embodiments, a process for preparing platelet rich plasma concentrate for hemostasis, at least about 97 volume percent of the hematocrit value of the blood and less than a major portion of the erythrocyte free platelet rich plasma can remain after the erythrocyte separation is retained by the depth filter.

According to various embodiments, a process for preparing platelet rich plasma concentrate for all applications, at least about 99 volume percent of the hematocrit value of the blood and less than a major portion of the erythrocyte free platelet rich plasma can remain after the erythrocyte separation is retained by the depth filter.

According to various embodiments, a process for preparing platelet rich plasma concentrate for all applications, about 100 volume percent of the hematocrit value of the blood and less than a major portion of the erythrocyte free platelet rich plasma can remain after the erythrocyte separation is retained by the depth filter.

In the above processes for preparing platelet rich plasma concentrate for hemostasis, optionally less than a significant portion of the erythrocyte free platelet rich plasma remaining after the erythrocyte separation is retained by the depth filter.

A Platelet Rich Plasma separator assembly comprising a cylindrical outer wall closed at the top by an upper plate and closed at the bottom. The outer wall has an inner surface; a cylindrical inner wall concentric with the cylindrical outer wall and having a top edge and a bottom. The inner wall has a central axis. The bottom of the inner wall is closed by a sloped bottom plate having a central opening, the bottom plate having an upper surface sloped down to a central opening. The top edge of the inner wall terminates at a distance from the upper plate to define an annular erythrocyte passageway therebetween. The inner wall has an outer surface and an inner surface that slopes radially inward from its top edge to its bottom at an angle of from about 0.2 to about 5 degrees with the central axis of the inner wall. A cylindrical depth filter is positioned between the inner surface of the outer wall and the outer surface of the inner wall in communication with the inner wall through the erythrocyte passageway. The device can be combined with a PRP concentrator assembly. The concentration assembly has a PRP concentration sump; an axially concentric rigid stationary outlet tube secured to the housing and extending through the PRP separation assembly to the PRP concentrate sump; and the PRP separation assembly is attached to and positioned above the PRP concentration assembly to form a combined separator-concentrator assemblage that is rotatable about the outlet tube.

### BRIEF DESCRIPTION OF THE DRAWINGS

Fig. 1 is a cross-sectional view of a disposable separation and concentration assembly and a permanent drive assembly, with desiccating beads shown in only half of the concentration subassembly.
Fig. 2 is a front plan view of the outer housing of the separation-concentration assembly of this invention.
Fig. 3 is a perspective view of the outer housing of Fig. 2 showing details of the motor assembly connector.
Fig. 4 is a cross-sectional drawing of the separation-concentration sub-assemblies shown in Fig. 1.
Fig. 5 is a top view of the outer cap subassembly of the separation-concentration assembly shown in Fig. 4.
Fig. 6 is a cross-sectional view of the outer cap subassembly shown in Fig. 5, taken along the line 6-6.
Fig. 7 is an exploded, isometric view of the outer cap subassembly shown in Fig. 5.
Fig. 8 is a top view of the top bucket cap subassembly of the separation-concentration assembly shown in Fig. 4.
Fig. 9 is a cross-sectional view of the top bucket cap subassembly shown in Fig. 8, taken along the line 9-9.
Fig. 10 is an exploded view of the sample inlet subassembly.
Fig. 11 is a top plan view of the top bucket subassembly of the separation-concentration assembly shown in Fig. 4.
Fig. 12 is a cross-sectional view of the top bucket subassembly of Fig. 11, taken along the line 12-12.
Fig. 12A is a cross-sectional exploded sectional view of an upper portion of the separation-concentration sub-assemblies shown in Figs. 11 and 12.
Fig. 13 is a front plan view of the valve assembly of the separation-concentration assembly shown in Fig. 4.
Fig. 14 is an exploded, isometric view of the valve assembly of Fig. 13.
Fig. 15 is a cross-sectional view of the bottom bucket subassembly shown in Fig. 4, taken along the central axis.
Fig. 16 is an enlarged cross-sectional view of the motor drive connector shown in Fig. 15.
Fig. 17 is a front plan view of the basket subassembly of the separation-concentration assembly shown in Fig. 4.
Fig. 18 is a cross-sectional view of the basket subassembly of Fig. 16, taken along the line 18-18.
Fig. 19 is a top plan view of the mixer assembly of the separation-concentration assembly shown in Fig. 4.
Fig. 20 is a cross-sectional view of the mixer assembly of Fig. 19, taken along the line 20-20.
Fig. 21 is an isometric view of the mixer assembly of Figs. 19 and 20.
Fig. 22 is a perspective view of the motor drive assembly of this invention.
Fig. 23 is a cross-sectional view of the motor drive assembly of Fig. 22 taken along the line 23-23.
Fig. 24 is a cross-sectional view of the motor drive assembly of Fig. 22 taken along the line 24-24.
Fig. 25 is a cross-sectional view of the upper bucket and valve assembly of Fig. 4, taken along the central axis.
Fig. 26 is a cross-sectional view of the upper bucket and valve assembly of Fig. 21, taken along the line 26-26.
Fig. 27 is a cross-sectional view of the upper bucket and valve assembly of Fig. 4, after the centrifugal action of the spinning upper bucket has extended the arms of the valve assembly and opened the valve.
Fig. 28 is a cross-sectional view of the view of upper bucket and valve assembly of Fig. 27, taken along the line 28-28.
Fig. 29 is a cross-sectional view of the upper bucket and valve assembly of Fig. 27, after rotational displacement of the arms of the valve assembly.
Fig. 30 is a cross-sectional view of the upper bucket and valve assembly of Fig. 29, taken along the line 30-30.
Fig. 31 is a cross-sectional view of the upper bucket and valve assembly of Fig. 29, after centrifugal separation has been completed.
Fig. 32 is a cross-sectional view of the separation and concentration assembly of Fig. 1, after blood has been introduced into the separation chamber.
Fig. 33 is a cross-sectional view of the separation and concentration assembly of Fig. 32 as erythrocytes are separated from the plasma-platelet mixture during high speed centrifugation.
Fig. 34 is cross-sectional view of the separation and concentration assembly of Fig. 33, after platelet-plasma fraction has passed into the concentration chamber.
Fig. 35 is a cross-sectional view of the separation and concentration assembly of Fig. 34 at the beginning of the high speed centrifugation to separate the platelet-plasma concentrate from the hydrogel bead.
Fig. 36 is a cross a cross-sectional view of the separation and concentration assembly of Fig. 35 after platelet-plasma concentrate has collected in the platelet-plasma concentrate sump.

### DETAILED DESCRIPTION

The apparatus and method can prepare a PRP concentrate that combines enhanced platelet levels in a plasma concentrate, in which the fibrinogen levels have not been significantly denatured. The product can combine the sealant and properties of the plasma concentrates for use in certain types of surgery with the healing properties provided by elevated platelet levels.

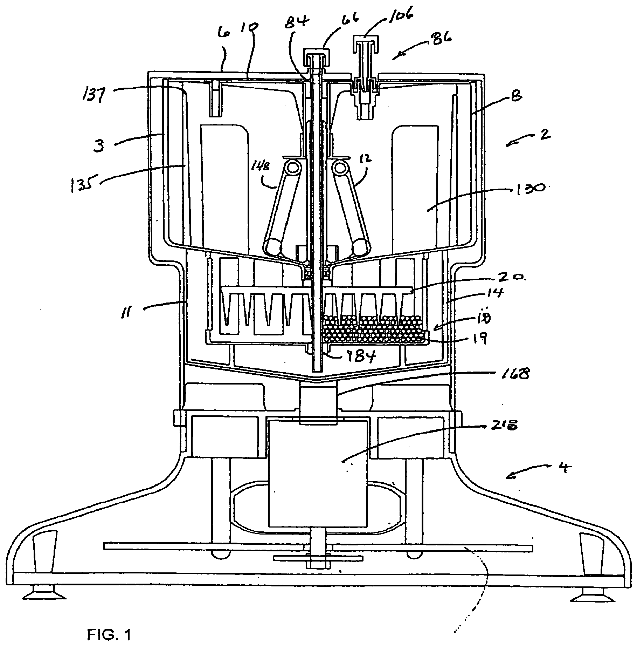

Fig. 1 is a cross-sectional view of a disposable separation and concentration assembly and a permanent drive assembly, with desiccating beads shown in half of the concentration subassembly. Details of the subsections of this assembly are hereinafter described in conjunction with more detailed drawings.

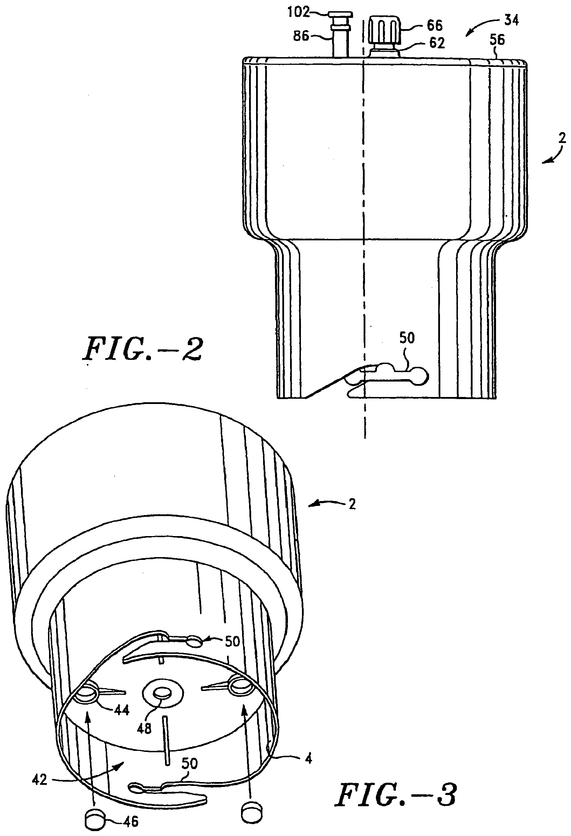

The upper housing 2 is described in greater detail hereinbelow in conjunction with Figs. 2 and 3.

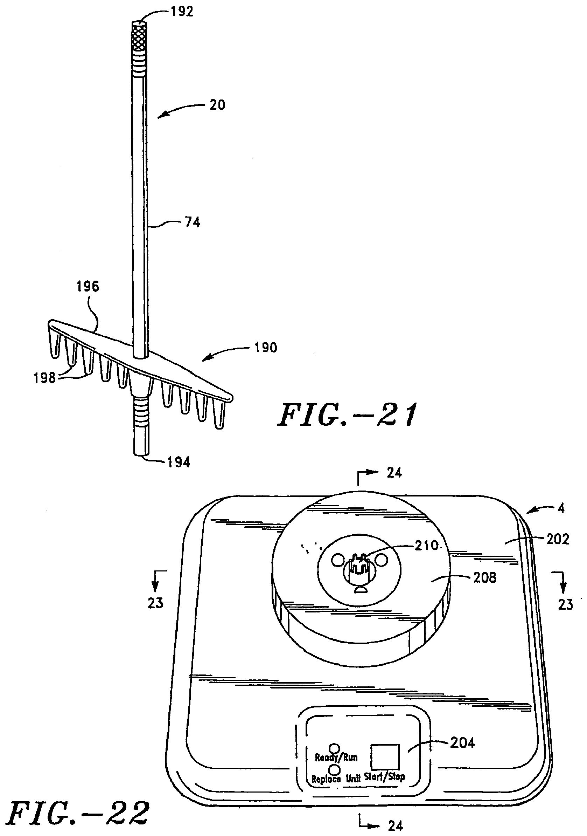

The motor drive subsystem 4 is described together with the motor drive system in conjunction with Figs. 22-24.

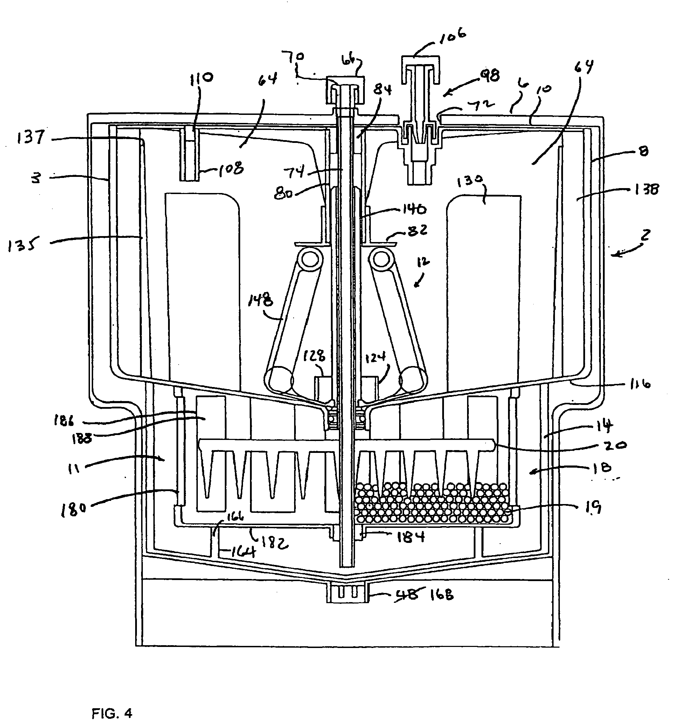

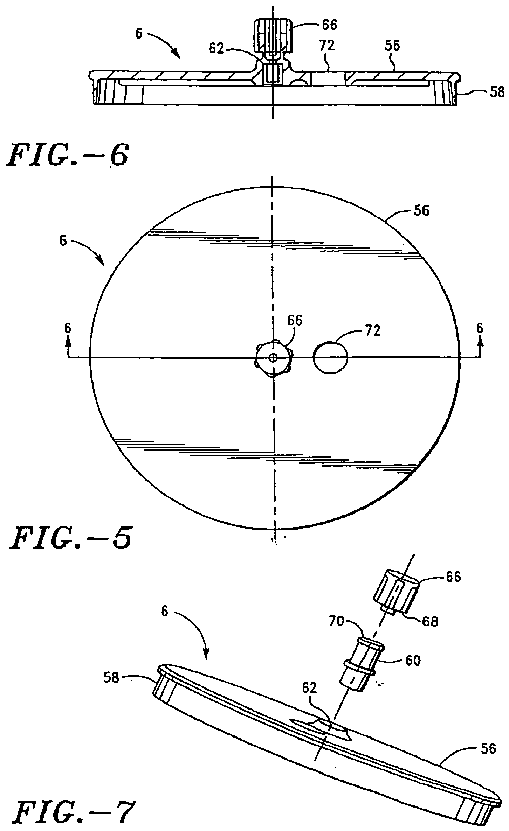

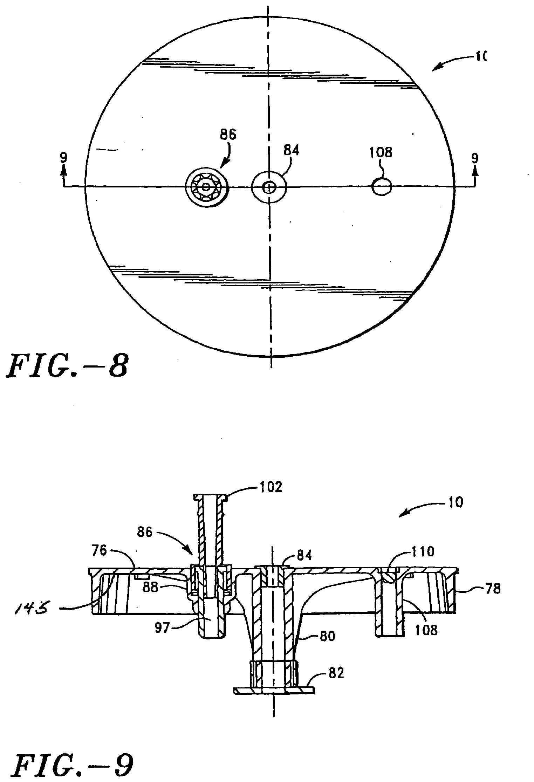

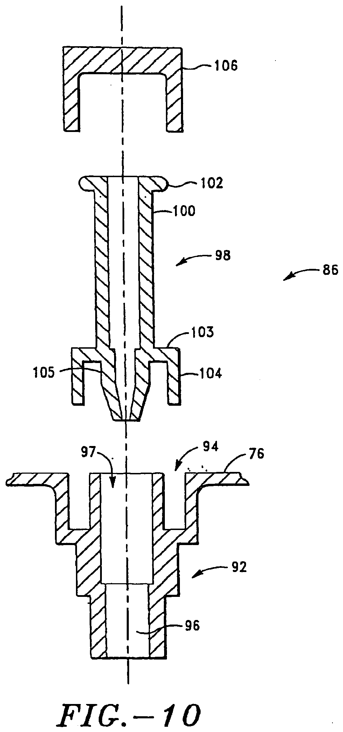

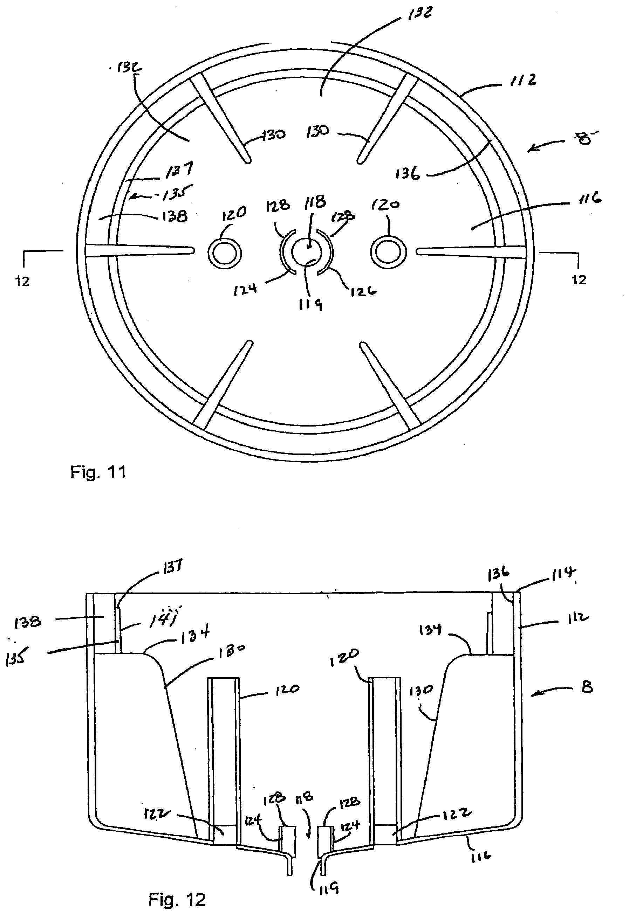

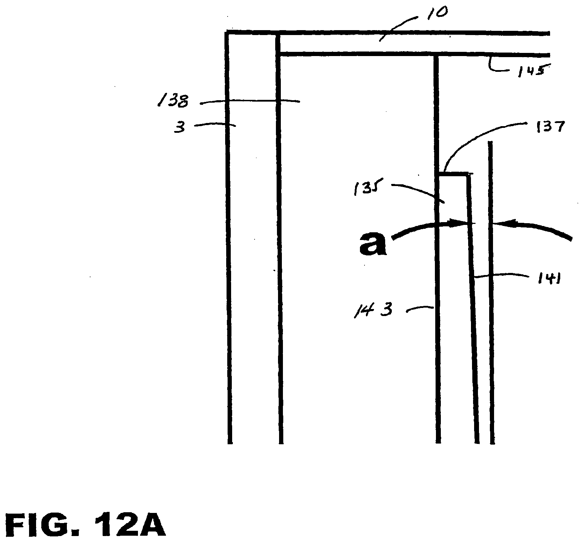

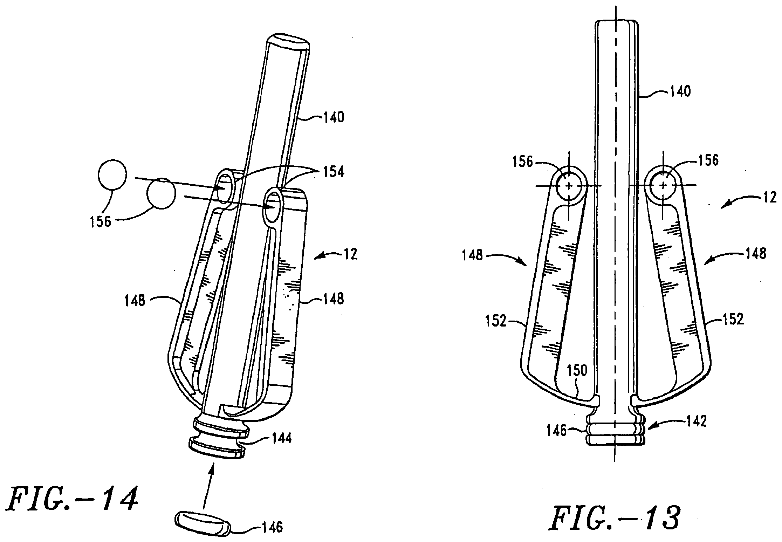

The separation system 3 enclosed in the upper housing 2 is described in greater detail with regard to Fig. 4. The separation system comprises a combination of subsystems including the outer cap subassembly 6 described in greater detail with respect to Figs. 5-7; a top bucket 8 described in greater detail with regard to Figs. 8 and 9; a sample inlet subassembly shown in Fig. 10; a top bucket cap subassembly 10 described in greater detail with respect to Figs. 11 and 12; and a valve subassembly 12 described in greater detail with respect to Figs. 13 and 14.

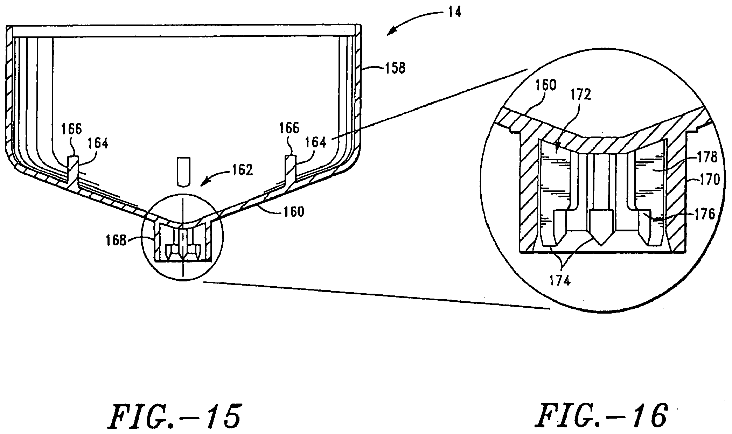

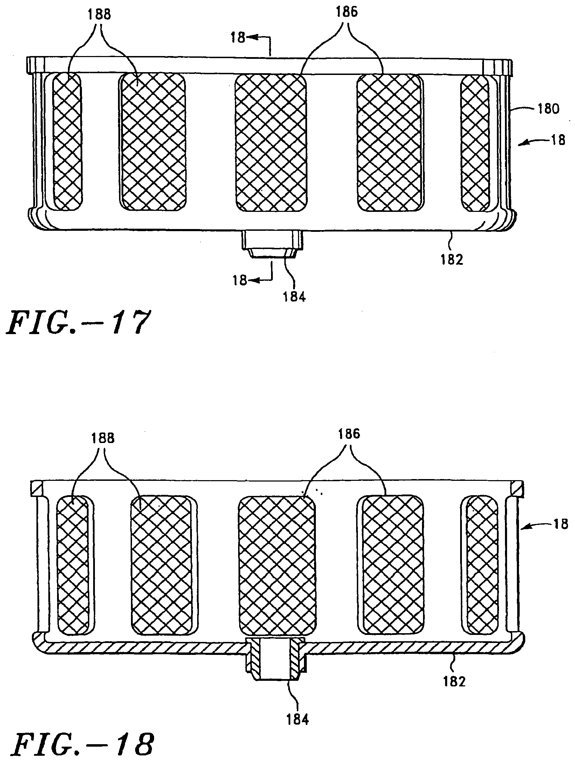

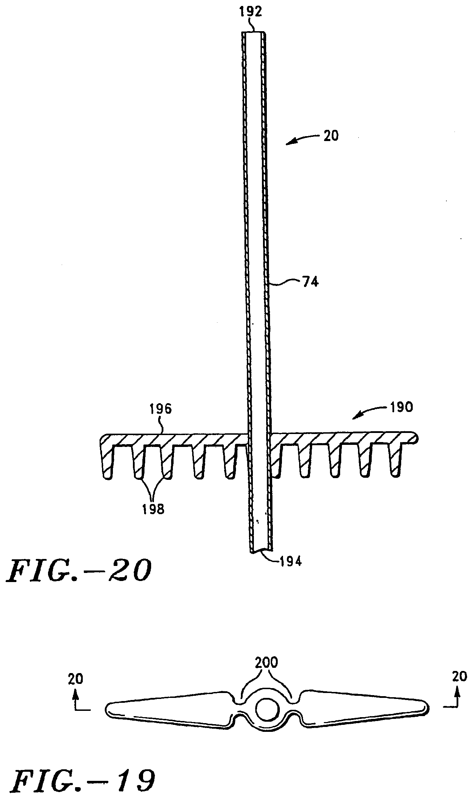

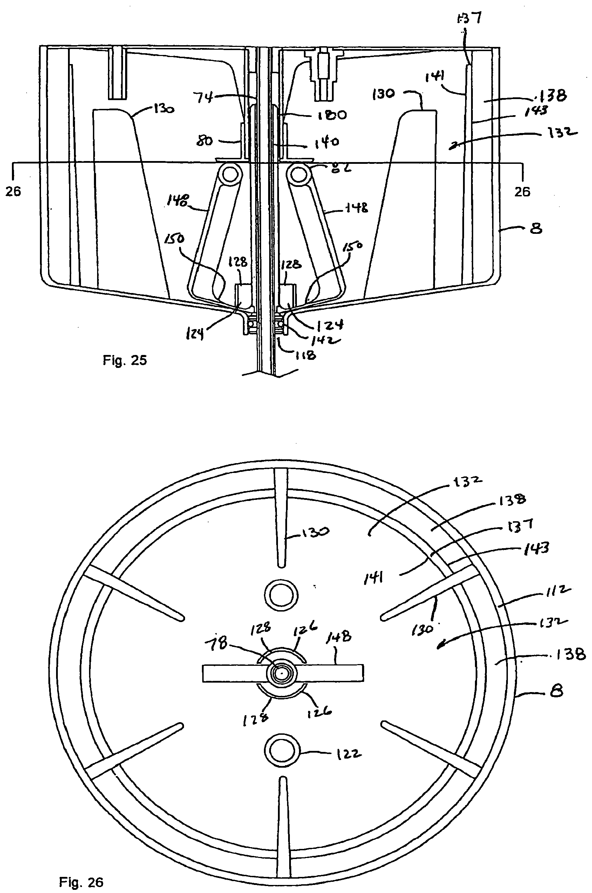

The concentrating system 11 includes a lower bucket 14 and drive connector 16, described in greater detail with regard to Figs. 15 and 26; a basket subassembly 18 described in greater detail with regard to Figs. 17 and 18; and a mixer assembly described in greater detail with regard to Figs. 19 to 21.

Fig. 2 is a front view of the outer housing of the separation-concentration assembly of this invention, and Fig. 3 is a perspective view of the outer housing of Fig. 2 showing details of the motor assembly connector.

The upper housing 2 isolates the sterile separation and concentration systems shown in Fig. 1. The upper portion of the outer housing 2 is sealed with an outer cap subassembly 34 having a blood inlet tube 86 and a PRP concentrate outlet port 62 and cap 66. Referring to Fig. 3, the lower assembly connector has a drive recess 42 shaped to engage the motor subassembly, and with spacer receptors 44 for holding spacers 46. The outer housing 2 and its enclosed separation components are a disposable unit and can be used with a permanent drive assembly shown in Figs. 1 and 22 to 24. The lower assembly includes an axially concentric motor drive receptor 48 and a plurality of tapered engagement and locking slots 50 that engage with corresponding mounting projections of the motor drive assembly (not shown).

Fig. 4 is a cross-sectional drawing of the separation-concentration sub-assemblies shown in Fig. 1. The outer housing 2 encloses an upper separation subassembly 3 and a lower concentration subassembly 11.

The top of the outer housing 2 is closed with outer cap subassembly 6 shown in greater detail with regard to Figs. 5-7. The outer cap subassembly 6 comprises a circular cap 56 with an annular flange 58 extending downward for securing it to the top of the upper housing 2. Concentrate outlet conduit 60 passes through an outlet conduit hole 62 in the center of the plate 56, extending through the plate and communicating with the separation chamber 64 (Fig. 4). Circular cap 66 has a central receptor 68 that engages with a Luer fitting 70 on the upper end of the outlet conduit 60 to maintain a sterile closure during the separation process.

An inlet port hole 72 is positioned in the circular cap 56, spaced from the central axis. The inlet port hole 72 is sized to engage the exterior inlet conduit 74 shown in Fig. 4.

The Luer fitting 70 is provided to engage an empty applicator syringe for removing platelet rich plasma concentrate product according to this invention. The lower end of the concentrate outlet conduit 60 constitutes a receptor for receiving the upper end of rigid tube 74 (Fig. 4).

The bucket cap 10 shown in Fig. 4 is described in greater detail with regard to Figs. 8 -10. Fig. 8 is a top view of the top bucket cap subassembly 10 of the separation-concentration assembly shown in Fig. 4, and Fig. 9 is a cross-sectional view of the top bucket cap subassembly shown in Fig. 10, taken along the line 9-9. The cap subassembly 10 closes the top separation bucket 8 shown in greater detail with respect to Figs. 11 and 12. The top bucket cap 10 comprises a circular plate 76 with a connecting flange 78 that extends downward from the lower edge of plate 76. The upper plate 6 is fixed to the outer housing 2 (Fig. 4) and is stationary during the separation and concentration processes. Top bucket cap 10 is secured to the top bucket 8 during the separation and concentration processes.

The circular cap 10 has an axially concentric hole with a valve assembly guide tube 80 extending downwardly therefrom. The lower end of the guide tube 80 has a valve assembly stop flange 82 secured thereto. The upper end of the guide tube 80 supports sleeve bearing 84. The sleeve bearing 84 can be formed of materials that are wear resistant and can be sterilized in an appropriate manner. Materials that can be used for the sleeve bearing include polyaryletherketone such as TECAPEEK™ Classix™, and the like.

The circular cap 10 has a sample inlet subassembly 86 that aligns with the hole 72 in the circular cap 56 (Fig. 5).

Fig. 10 is an exploded view of the sample inlet subassembly 86. The sample inlet subassembly 86 comprises an inlet tube 92 mounted in the plate 76, the top of the inlet tube 92 including an annular receptor 94. A sterile filter 96 can be positioned in the lower end of the passageway 97 of tube 92.

The subassembly 86 includes a removable inlet tube 98. Inlet tube 98 comprises a central tube 100 having at its upper end an integral Luer fitting 102. At an intermediate level of the tube 100, an annular plate 103 extends outward from the tube 100. An integral cylindrical flange 104 extends downward from the outer edge of the plate 103. The flange 104 is sized to engage the receptor 94. The lower end 105 of the tube 100 is sized to engage the upper end of the passageway 97.

The inlet tube is provided with a cap 106 that engages the Luer fitting 102 to provide a sterile closure of the removable inlet tube 98 prior to use, such as during shipment and handling.

The inlet tube 98 in passing through the hole 72 in the stationary circular cap 56 locks the separation and concentration subassemblies against rotation during shipment and storage. After the patient blood is introduced into the top bucket 8 (Fig. 4) through the inlet subassembly 86, the inlet tube 98 is removed, unlocking the separation and concentration sub-assemblies 3 and 11 from the stationary circular cap 6, freeing them for rotation about the central tube 74.

A sterile breathing tube 108 is secured to the circular plate 76 to permit air flow from the separation chamber 64 when blood is introduced and to permit air movement into the system when platelet-rich concentrate is removed from the concentrating system 11, as described in greater detail hereinafter. Sterile air filter 110 in breathing tube 108 (Fig. 9) prevents entrance of micro-organisms into the interior of the separation chamber, preserving sterility.

The top bucket subassembly in Fig. 4 is shown in detail in Figs. 11 and 12. Fig. 11 is a top view of the top bucket subassembly 10 of the separation-concentration assembly shown in Fig. 4, and Fig. 12 is a cross-sectional view of the top bucket subassembly of Fig. 11, taken along the line 12-12. The top bucket subassembly 10 comprises a cylindrical outer wall 112 having a top edge 114 that is secured to the inner surface of the flange 58 of the upper bucket cap 10. The lower end of the cylindrical outer wall 112 is closed with integral sloped floor plate 116 with a central passageway 118 that constitutes a central flow passageway for separated platelet-plasma. The inner wall surface of the passageway 118 constitutes a valve seat 119 for the valve assembly described in greater detail hereinafter with respect to Figs. 13 and 14. Spaced from the central passageway 118 and secured to the floor plate 116 are vent columns 120 with filters 122 in their bottom. The columns 120 serve as vents allowing movement of air from the concentration subassembly into the separation chamber when liquid flows through downward through the central passage 118, as is explained hereinafter. Filters 122 prevent escape of hydrogel beads from the basket subassembly 18 through the vent columns 120 during transport or handling of the device of this invention. Surrounding the central passageway 118 and secured to the upper surface of the tapered floor plate 116 are upwardly extending abutment plates 124, each having an upper valve arm abutment surface 128.

Referring to Fig. 12A, the separation zone can be further limited by an inner cylinder 135 extending from the upper surface of the bottom plate 116 upward to an upper lip 137. The inner surface of the cylinder 141 can have a slope "a" of between about 0.2 to about 5 degrees and optionally between about 0.2 to about 2 degrees relative to the central axis. This slope can be selected to facilitate flow of erythrocytes along the surface and beyond the lip 137 into the depth filter 138. Because the slope can be selected to be small, the separation of platelets trapped by the erythrocytes is facilitated, leaving a maximum quantity of platelets suspended in the plasma phase.

The depth filter 138 can extend from the bottom surface 145 past top edge 137 to completely block the opening defined by the top lip 137 and the bottom surface 145. This can be provided to assist in preventing the return of erythrocytes to the plasma from the volume between the outer cylinder 3 and the inner cylinder 135. In various embodiments, the depth filter can extend to the bottom plate 116 which can insure complete capture of erythrocytes. In various embodiments, if the space between the upper edge 137 and lower surface 145 is sufficiently small and risk of a return of a small amount of erythrocytes to the plasma is acceptable, the depth filter can be omitted.

A plurality of radially inwardly extending separation plates 130 are secured to the inner surface of the cylindrical outer wall 112 and the sloped floor plate 116. Each adjacent pair of these plates defines a separation zone 132. The plates 130 can be evenly spaced around the cylindrical outer wall to provide a balanced subassembly. They can be in matched, opposed pairs, for example the three matched sets as shown in Fig. 11. The top edge 134 of each of the separation plates 130 is spaced at a distance below the top edge 114 to permit overflow of blood in order to achieve an even distribution of blood between each the separation zones 132 during the spin acceleration stages and during the spin deceleration stages. This can maintain balance and minimize vibration of the rotating assembly.

The space between the interior surface 136 of the cylindrical outer wall segments and the outer surface 143 of the inner cylinder in each of the each separation zones 132 contains the open-cell foam segment or depth filter segment 138. The foam segments 138 have pores and passageways sized to allow infiltration of erythrocytes into the foam and subsequent entrapment of erythrocytes during the high speed centrifugation of the separation stage. The pores and passageways are sized to retain entrapped erythrocytes thereafter when the spinning slows or stops and the erythrocyte-free platelet-plasma suspension flows downward through the opening 118.

The distance between the lip 137 and the lower surface 145 of the upper plate is sufficient to permit flow of all erythrocytes into the depth filter 138 during the separation phase. It can be between about 0.02 and about 50 mm, such as about 0.02 mm to about 10 mm, or another dimension that facilitates a selected amount, such as complete erythrocyte removal while trapping a minimum portion of the platelets.

The provision of the inner sloped cylinder can increase the proportion of platelets remaining in the plasma.

Fig. 13 is a front view of the valve assembly 12 of the separation-concentration assembly shown in Fig. 4, and Fig. 14 is an exploded, isometric view of the valve assembly of Fig. 13. The valve assembly 12 comprises a central tube 140, the lower end constituting a valve face 142. The valve face 142 comprises an annular receptor 144 that receives and holds an O-ring 146. The outermost surface of the O-ring 146 can be sized to form a sealing engagement with the valve seat 119 (See Figs. 11 and 12).

The valve assembly 12 includes two opposed centrifugal arms 148 secured to the tube 140 above the valve face 142. Each centrifugal arm 148 has a flexible portion 150 adjacent the tube 140 and a rigid arm portion 152. The distal end of the rigid arm portion 152 includes a weight receptor 154 in which a weight 156 is secured to provide additional weight to the end of the rigid arm portion. Operation of the valve assembly is described hereinafter with respect to Figs. 25-31.

The lower bucket 14 in Fig. 4 is shown in detail in Figs. 15 and 16. Referring to Fig. 15, the lower bucket 14 has a cylindrical sidewall 158 and a sloped bucket bottom 160, the lower portion of which forms a platelet-plasma concentrate sump 162 in which concentrated platelet and plasma concentrate collects. A plurality of basket supports 164 extend upward from the top surface of the slopped bucket bottom 160, the top surfaces 166 of which support a concentrating basket subassembly 18 described hereinafter with regard to Figs. 17 and 18.

An axially concentric drive receptor 168, shown in detail in Fig. 16, is secured to the bottom surface of the bucket bottom 160. The drive connector receptor 168 can have any configuration that will releasably couple with a suitably configured motor drive connector. In the configuration shown in Figs. 15 and 16, the drive receptor 168 comprises an outer cylinder 170 and a plurality of ridges 172, each ridge having a tapered leading engagement surface 174, an abutment surface 176 and an upper plate 178. The upper plate 178 transmits the torque from the drive motor (described hereinafter with respect to Figs. 22-24) to the lower bucket bottom 160 and from there to the concentrating and separating subassemblies, all of which are secured together to form a unitary rotatable assembly.

Fig. 17 is a front view of the basket subassembly 18 of the separation-concentration assembly shown in Fig. 4, and Fig. 18 is a cross-sectional view of the basket subassembly of Fig. 17, taken along the line 18-18. The basket subassembly 18 comprises a cylinder 180 secured to a circular floor plate 182. A slip bearing 184 is positioned in the axial center of the circular plate 182 for engaging the rigid tube 74 (Fig. 4). The slip bearing 184 can be formed of a material that is wear resistant and can be sterilized in an appropriate manner, these materials can be the same or similar to those that form the sleeve bearing 84. The cylinder 180 has an array of windows or openings 186 around its circumference, each window closed or coveted with a fine screen 188 having a mesh size sufficiently small to prevent escape of hydrogel beads 19 (Figs. 1 and 4) from the basket during spinning.

Fig. 19 is a top view of the mixer assembly of the separation-concentration assembly shown in Fig. 4. Fig. 20 is a cross-sectional view of the mixer assembly of Fig. 18, taken along the line 20-20, and Fig. 21 is an isometric view of the mixer assembly of Figs. 19 and 20. The mixer assembly 20 comprises a rake 190 secured to stationary tube 74. The upper end 192 of the stationary tube 74 is secured to the upper cap subassembly 34 to secure it against rotation. At the lower end 194 of the stationary tube 74 is a port for removal of platelet-plasma concentrate from the sump 162 (Fig. 15). The rake 190 comprises a radially extending spine 196 from which integral rake elements 198 extend downward to an elevation short of the bottom plate 182 of the basket subassembly 18 as shown in Figs. 4, 17 and 18. The spine 196 can have optional breakaway notches 200 adjacent its center. The notches 200 weaken the spine and direct fracture of the spine 196 at the location of the notches in the event that the pressure produced by contact of the beads 19 with the rake elements 198 during the final centrifugal spin becomes excessive.

The stationary tube 74 extends through the sleeve bearing 184 of the basket subassembly 18 and through the sleeve bearing 84 of the top bucket cap, permitting free rotation of the separating and concentrating assemblies around the stationary tube. The stationary tube 74 is fixed to the outer cap subassembly 6 and the stationary outer housing 2.

Fig. 4 is a comprehensive assemblage of the components shown in Figs. 5-20.

Concentrating desiccating hydrogel beads 19 fill the lower half of the basket 18 (only one side is shown empty to enable unobstructed viewing of the windows 186 and screen 188 elements (Figs. 1, 17 and 18).

The concentrating desiccating hydrogel beads 19 can be insoluble beads or disks that will absorb a substantial volume of water and low molecular weight solutes while excluding high molecular weight solutes and particulates and will not introduce undesirable contaminants into the plasma. They can be dextranomer or acrylamide beads that are commercially available (appropriate materials include Debrisan from Pharmacia and BIO-GEL P™ from Bio-Rad Laboratories, respectively). Alternatively, other concentrators can be used, such as SEPHADEX™ moisture or water absorbents (available from Pharmacia), silica gel, zeolites, cross-linked agarose, etc., in the form of insoluble inert beads.

Fig. 4 in conjunction with subassembly Figs. 5-21 shows the assembly prior to use with the valve assembly 12 secured for shipment by the sleeve 80 into which the valve assembly tube 140 extends and the abutment flange 82 is secured to the bottom of the sleeve 80. The valve face 142 is shown in position against the seat 119. This confines the beads to the basket 18 and prevents escape of beads into the upper separation chamber 64 if the device is inverted or shaken during transport or handling.

The assembly is secured against rotation around the rigid tube 74 by the position of the removable inlet tube 98 in the hole 72 of the stationary outer cap subassembly 6.

The upper edge of the cylinder 180 of the basket assembly 18 is secured against the lower surface of the tapered bottom 116, and the lower surface of the plate 182 is secured against the upper edge surfaces 166 (Fig. 15) of the supports 164.

Thus assembled, the upper separation subassembly 3 and the lower concentration subassembly 11 rotate as a single unit around the fixed tube 74. The upper separation subassembly is positioned on the central tube 74 by the slip bearing 84 through which the fixed tube 74 extends. The lower separation subassembly is positioned on the central tube 74 by the slip bearing 184 through which the fixed tube extends. The rake assembly 20 including the tube 74 remains stationary during rotation of the separation and concentration subassemblies 3 and 11 in the separation and concentration phases, to be described in greater detail hereinafter.

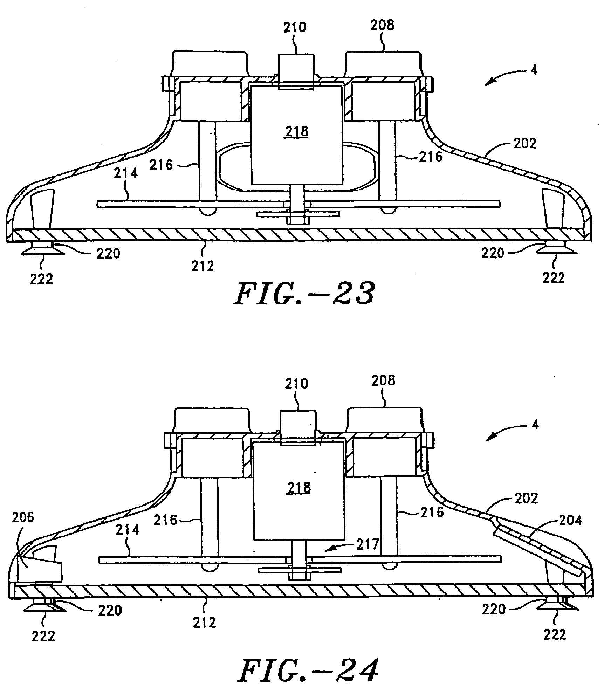

Fig. 22 is a perspective view of the motor drive assembly. Fig. 23 is a cross-sectional view of the motor drive assembly taken along the line 23-23, and Fig. 24 is a cross-sectional view of the motor drive assembly taken along the line 24-24.

The outer shell 202 of the motor housing 4 encloses the motor 218 and supports the control interface 204 and the power connector 206. The separation-concentrating assemblies are supported on the raised annular support surface 208 surrounding the motor connector 210. Motor connector 210 has a configuration that will releasably engage the drive receptor 168 (Fig. 16). The bottom of the housing 22 is closed by support plate 212. A control and power plate 214 for the system is supported by four support struts 216 attached to the underside of the housing shell 202. Plate 214 is a conventional printed circuit or equivalent board with the electronic components of the control and power system for the device, and in its center, a support 217 for the motor 218. The electrical components are connected to the control interface 204 and power connector 206 by conventional wiring circuits (not shown). Four support feet 220 are secured to the bottom of the support plate 212 and provide friction surfaces 222 to secure the device on a laboratory surface.

Figs. 25-31 illustrate the operation of the valve subassembly during and immediately after the initial separation process. Blood and blood products are omitted from these cross-sectional views to allow an unobstructed view of the valve assembly elements at each stage.

Fig. 25 is a cross-sectional view of the upper bucket and valve subassembly of Fig. 4, taken along the central axis, and Fig. 26 is a cross-sectional view of the upper bucket and valve assembly of Fig. 25, taken along the line 26-26. This is the view when blood is initially introduced into the top bucket 8. The arms 148 of the valve subassembly are in their initial upright position, with the central tube 140 positioned in the guide tube 80 and the upper end of each arm contacting the flange 82. The valve face 142 is in position in the valve seat 119 (Fig. 12) at the upper end of the central passageway 118, closing the passageway and preventing escape of blood. The flexible portions 150 of the arms 148 are positioned in the channels between the abutment plates 124 and 126, preventing rotation of the arms 148 about tube 74 during shipment and handling.

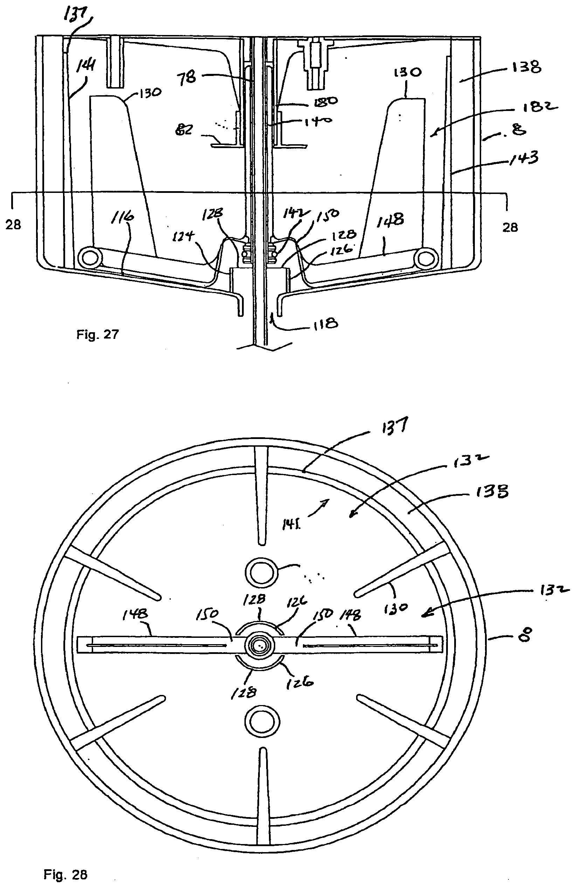

Fig. 27 is a cross-sectional view of the upper bucket and valve assembly of Figs. 25 and 26, after the centrifugal action of the spinning upper bucket has extended the arms of the valve assembly and opened the valve, and Fig. 28 is a cross-sectional view of the view of upper bucket and valve assembly of Fig. 27, taken along the line 28-28. After the desired volume of patient blood has been introduced into the top bucket 8, the separation and concentration assembly is rotated around the tube 74 at a high speed, the centrifugal force created by this rotation causing the blood to flow outward where it can be distributed evenly by the separation plates into the separation zones 132. The centrifugal force pools the blood against the outer surface of the foam segments 138 where the more dense materials, such as erythrocytes, preferentially move into the foam, leaving behind less dense material, such as erythrocyte-free plasma which can also contain the less dense platelets.

Under the force of centrifugation, the valve arms 148 rotate outward until they contact the sloped floor 116. This action slides the valve central tube 140 upward to the upper portion of the guide cylinder 180, pulling the valve face 142 from the central passageway 118 and out of contact with the valve seat 119 to open the passageway 118. As the arms 148 rotate outward and the valve face 142 is lifted, the lower flexible ends 150 of the arms 148 are also pivoted upward from between the abutment plates 124 and 126, freeing the arms for rotation about the tube 74. Because the liquid is held against the foam segments 138 by centrifugal force, it does not flow through the open passageway 118.

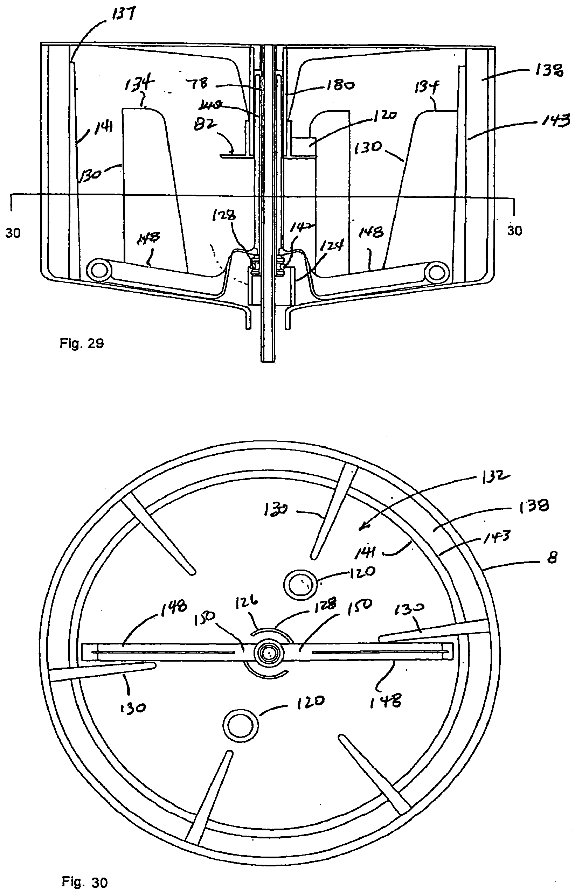

Fig. 29 is a cross-sectional view of the upper bucket and valve assembly of Figs. 27 and 28, after rotational displacement of the arms of the valve assembly, and Fig. 30 is a cross-sectional view of the valve structure of Fig. 29, taken along the line 30-30. When the arms 148 are lifted from between the abutment plates and are freed from constraint by the abutment plates 124, rotational motion causes the arms 148 to rotate about the rigid tube 74. The rotation continues until one of the arms 148 contacts an adjacent separation plate 130 in its rotational path. This rotational displacement aligns the lower flexible ends 150 of the arms 148 above a portion of an abutment surface 128 of an abutment plate 124.

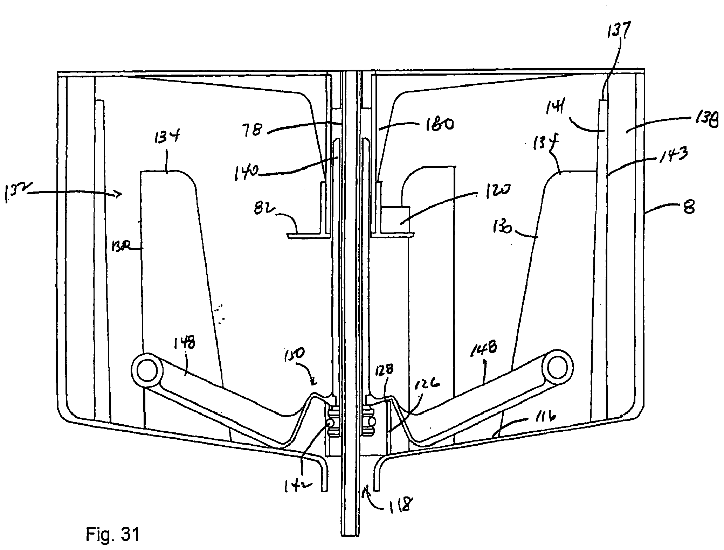

Fig. 31 is a cross-sectional view of the upper bucket and valve assembly of Fig. 29 and 30, after centrifugal separation has been completed and the rotation of the separation and concentration subassemblies is slowed or stopped. Under the force of gravity, the platelet-plasma mixture flows to the bottom of the tapered floor 116, down its sloped surface to the central passageway 118, and through the central passageway 118 to the basket subassembly 18 for concentration. The removal of the strong centrifugal action may permit the arms 148 to spring upward, causing the valve face 142 to move downward toward the central passageway 118. This movement is stopped when one or both flexible arm portions 150 contact an opposed abutment surface 128, leaving the central passageway open to the flow of the platelet-plasma mixture.

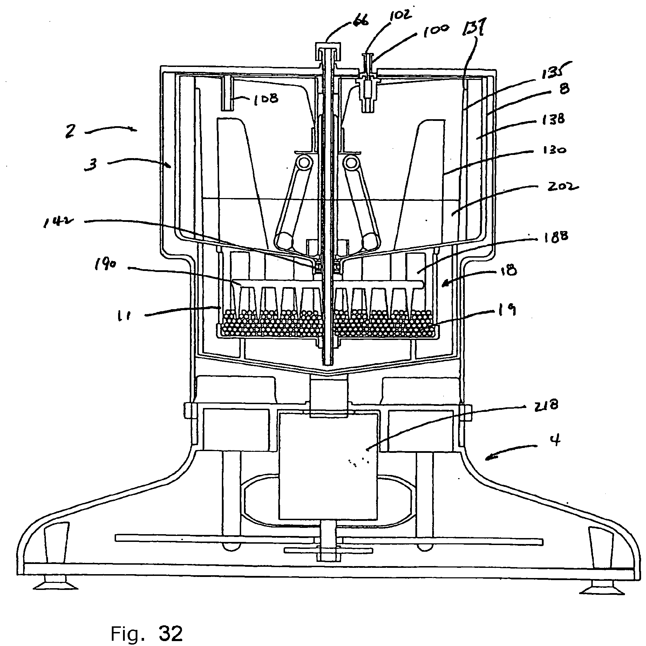

The operation of the device including the separation phase and concentrating phase of a material, such as whole blood, are described hereinafter in conjunction with Figs. 32-36. Although the separation and concentration of whole blood are described in detail, other materials can also be separated and concentrated.

Fig. 32 is a cross-sectional view of the separation and concentration assemblies of Fig. 4, after blood 202 has been introduced into the separation assembly 3 through the tube 110 from a syringe secured to the Luer fitting 102. The upper tube 100 with the Luer fitting is then removed, unlocking the separation and concentration assemblies 3 and 11 for rotation. The blood flows into the bottom of the top bucket 8. Air displaced by the incoming liquid escapes through breathing tube 108. The valve face 142 is in a closed position, preventing escape of the blood from the bucket 8. The operation of the system is then initiated, and the motor 218 spins the separation and concentration assemblies together around the rigid tube 74.

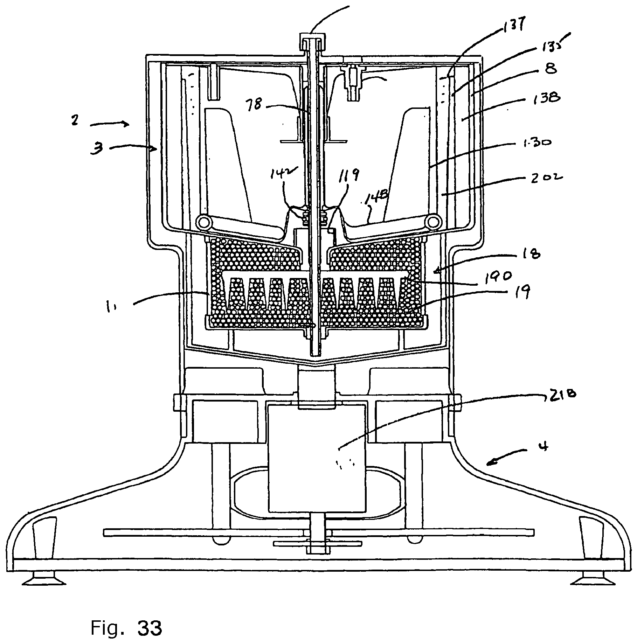

Fig. 33 is a cross-sectional view of the separation and concentration assemblies of Fig. 32 as erythrocytes are separated from the plasma-platelet mixture during high speed centrifugation. As the separation and concentration assemblies turn at a high speed, the blood is forced against the foam 138. The erythrocytes, being denser than other blood components, migrate into the pores and passageways of the foam. The valve subassembly opens the valve 142 as the centrifugal forces pivot the outer ends of the arms 148 away from the center, raising the valve face 142 face from valve seat 119 in the central passageway 118. However, as long as the high speed centrifugation continues, all of the liquid can be maintained against the foam. The centrifugal forces also force the hydrogel beads 19 radially outward against the outer screens 188 of the basket subassembly, out of contact with elements of the rake 190. Centrifugation is continued until a majority of the erythrocytes are completely trapped in the foam. Because any erythrocytes can weaken the gel product formed when the product is applied, the removal of a maximum proportion of the erythrocytes can be selected. The speed of centrifugation tends to separate erythrocytes from platelets, leaving a substantial portion of the platelets in the plasma while entrapping a majority of the erythrocytes in the foam.

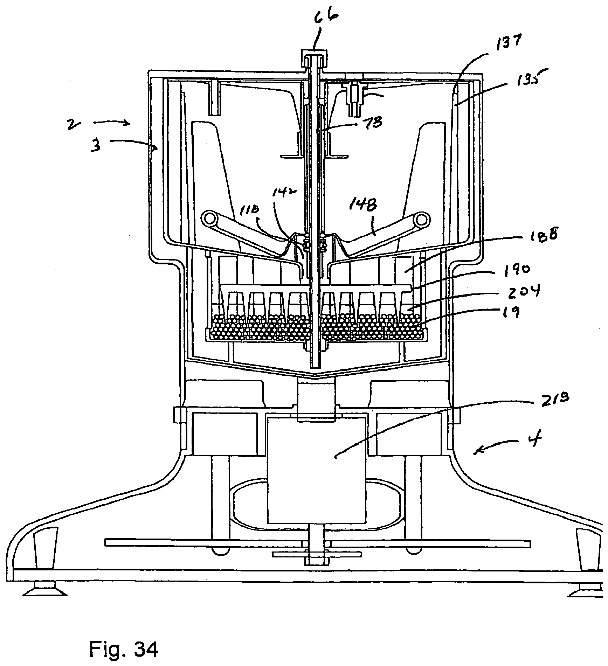

Fig. 34 is a cross-sectional view of the separation and concentration assembly of Fig. 33. After the spinning is slowed or stopped, the platelet-plasma fraction 204 flows to the bottom of the upper bucket 8 and down through the central passageway 118 into the basket subassembly 18 where it comes into contact with the desiccating hydrogel beads 19. These beads concentrate the plasma by absorbing water from the liquid. The separation and concentrating assemblies are then rotated at a slow speed by the motor 218, stirring the beads by moving them through the stationary spines 196 of the rake 190. Agitating the beads insures maximum contact of the beads surfaces with the plasma and reduces gel polarization that arises when the plasma thickens adjacent the bead surfaces. This desiccating phase is continued until the desired proportion of the water has been removed and the desired concentration of the plasma has been achieved.

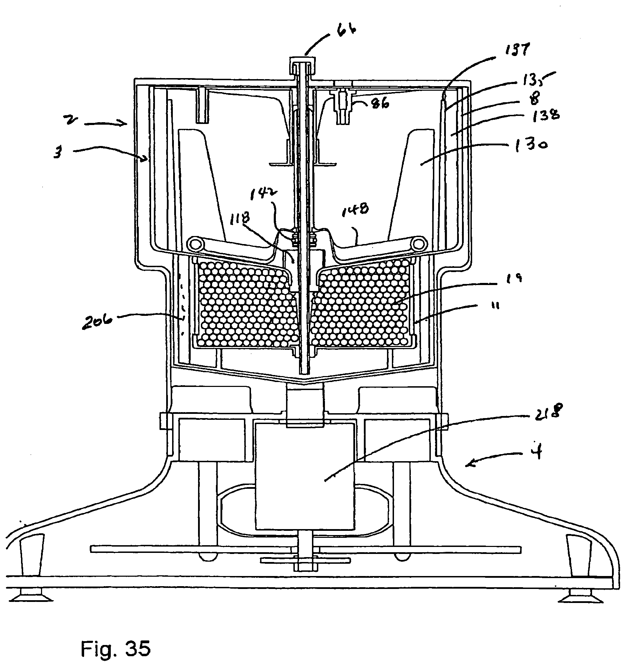

Fig. 35 is a cross-sectional view of the separation and concentration assembly of Fig. 34 at the beginning of high speed centrifugation separation of the platelet-plasma concentrate from the hydrogel beads. At this stage, removal and selected or maximum recovery of the platelet rich plasma concentrate 206 from the beads 19 is obtained. The separation and concentration assemblies are rapidly rotated by the motor 218 around the stationary tube 74, creating centrifugal forces that force the platelet rich plasma concentrate and the beads 19 against the screen elements 188 of the basket 18. The screen elements prevent escape of the beads 19 as the continuing centrifugal force causes the platelet enriched plasma concentrate to flow from the beads and through the screen. This high speed centrifugation is continued until a selected or maximum recovery of the platelet rich plasma is obtained.

The absorption of water by the hydrogel beads is accompanied by an increase in bead diameter, increasing the bead volume. If the increased bead volume causes the ends of the rake 190 to drag on beads packed on the screen surface, the rake can break along the break-away notches 200 (Fig. 19), and the rake fragments become mixed with the beads.

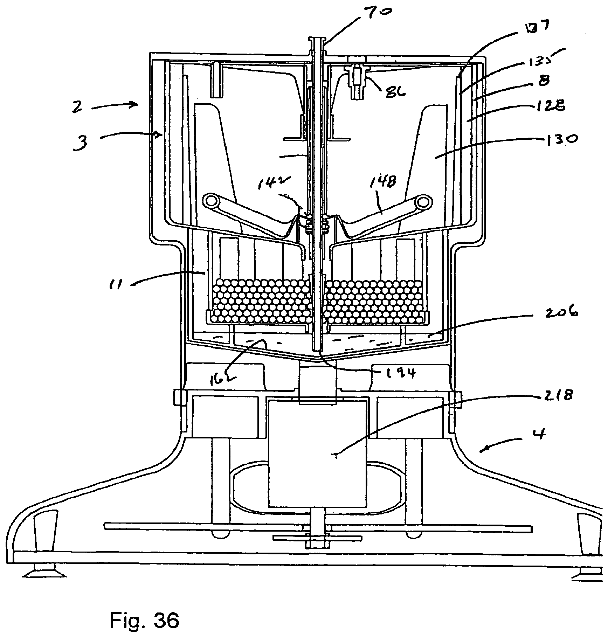

Fig. 36 is a cross-sectional view of the separation and concentration assembly of Fig. 35 after the high speed centrifugation has ended and the platelet-plasma concentrate has flowed into the platelet-plasma concentrate sump 162. The cap 66 has been removed, exposing Luer fitting 70 at the upper end of the tube 60. An applicator syringe (not shown) can be secured to the Luer fitting 70. The platelet-rich plasma concentrate is removed from the sump 162 by retracting the barrel of the applicator syringe, drawing platelet rich plasma concentrate up through the tubes 74 and 60 and into the syringe. Breathing tube 108 permits air to flow into the system to replace the volume of liquid removed by the syringe, thus preventing the creation of a partial vacuum in the system that would impede liquid removal.

Regarding the concentration factor, for maximum wound-healing, the platelet level can be maximized and high concentrate ion factors can be created. For homeostasis, plasma concentrations of about 3 to about 4 fold over anti-coagulated plasma levels are most effective. Concentrations below about 3 fold have an insufficient fibrinogen concentration. Concentrations higher than about 4 fold have excessive levels of total protein (principally albumin) which interferes with the fibrin gel structure. To obtain a preparation that maximizes haemostatic effectiveness while also providing improved (albeit perhaps less than maximal) wound-healing potential, a concentration range of about 3 to about 4 fold over anti-coagulated plasma levels may be selected. For applications where sealant activity is not desired, high concentrations may be selected.

Regarding erythrocyte levels, normal human hematocrits vary from about 37 percent or lower to about 52 percent for whole blood, measured after a very high speed spin. To achieve concentrations of about 3 fold or higher, some erythrocyte removal may be selected. However, the tensile strength of concentrated plasma gels diminish as the level of erythrocyte contamination increases. The concentration of erythrocytes in the final concentrate should be less than about 3 to about 5 percent to provide effective haemostatic properties. The separation and concentration device can be used to remove as much of the erythrocytes as is technically practical with the system, although trace contamination is acceptable. For applications where sealant activities are not desired, higher levels of erythrocytes can be maintained.

Regarding volume, both the depth filter and the beads reduce the liquid volumes being processed. Because of this volume loss, from about 14 to about 17 percent volume yields of effective haemostatic wound-healing product is generally obtained from average patient blood with the separation and concentration device. To make an effective product, the depth filter volume is selected to retain about 50 percent of the anti-coagulated blood (blood containing anticoagulant) and product about a 50 percent yield of PRP. The amount of the beads, in water absorption units, is selected to retain water equaling about 67 percent of the PRP volume.

Regarding accuracy, the amount of the depth filter and beads in each system is carefully selected to yield an optimum product. However, because of the wide range of hematocrit levels in patient populations, an approximate balance of components can be determined and selected.

If too much blood is added to the device, there is a greater chance that the product will have a substantial erythrocyte contamination, and the final product will be less concentrated than selected because the volume exceeds the practical capacity of the depth filter. Because the volume retained by the depth filter is about half the total volume of blood to be processed, if the volume of blood introduced into the device is too small, a substantially lower volume of PRP may be delivered to the beads. For example, if the blood volume is low by about 25 percent, this will result in about 50 percent of the desired volume being delivered to the beads. If the volume of PRP contacting the beads is low by 33 percent or more, no product will be recovered because the beads may absorb as much as about 67 percent or more of the targeted PRP volume. If the volume contacting the beads is short by about 17 percent, this may yield half of the desired volume of final product with twice the desired concentration (and hence of little value as a hemostat). In other words, a small error in the volume of blood introduced into the device is amplified into a large error in final product volume and concentration factor.

The systems can be designed to specifically match the hematocrit levels of the particular patient's blood to be processed. For a single optimized universal device, the device is optimized for the average patient blood, using fixed volumes of depth filter and blood, and a fixed bead water absorption capacity.

If it is selected to tolerate inaccuracy of introduced blood volume, the device can incorporate an overflow chamber as described in provisional patent application Serial No. 60/654,718 filed February 17, 2005 and U.S. Pat. application No. 11/342,761, filed on January 30, 2006.

### EXAMPLE

### Standard System Operation

Blood was processed with a device as shown and described in this application.
1) The initial spin was continued for 10 seconds at 250 rpm. This spin allows beads to be flung out into the cage under sufficiently low rpm that the initial imbalance does not generate excessive vibration. The outer ends of the rakes (the outermost tines) level the beads around the perimeter of the basket to balance the beads.
2) The erythrocytes were separated with the an erythrocyte separation spin of 3200 rpm for 90 seconds, packing the erythrocytes into the depth filter.
3) The PRP was concentrated by slowing the spin to 50 rpm for 45 seconds, draining PRP into the concentrator chamber and mixing the PRP with the beads.
4) The PRP concentrate was then removed from the beads by a final high-speed spin at 3200 rpm for 45 seconds.

The rates of acceleration and deceleration between stages were moderated to reduce vibration.
The process parameters were as follows:

| | |
|---|---|
| Start Volume | 150 cc |
| Retained by depth filter | 75 cc |
| Recovered concentrate | 23 cc |
| Platelet count | 3 fold increase over whole blood |
| Fibrinogen concentration | 2.8 - 3.2 fold increase over while blood |
| Erythrocytes in product | Undetected (less than 1 %) |

## Claims

1. A multi-component material separator assembly comprising:
a cylindrical outer wall (112) closed at the top by an upper plate (10) having a bottom surface (145) and closed at the bottom, the outer wall having an inner surface (136);
a cylindrical inner wall (135) concentric with the cylindrical outer wall (112) and having a top edge (137) and a bottom, the inner wall (135) having a central axis, the bottom of the inner wall being closed by a sloped bottom plate (116) having a central opening (118), the bottom plate (116) having an upper surface sloped down to a central opening (118), the top edge (137) of the inner wall (135) terminating at a distance from the upper plate (145) to define an annular erythrocyte passageway therebetween, the inner wall (135) having an outer surface (143) and having an inner surface (141) that slopes radially inward from its top edge (137) to its bottom at an angle from about 0.2 degrees relative to the central axis of the inner wall (135); and
a cylindrical depth filter (138) positioned between the inner surface (136) of the outer wall (112) and the outer surface (143) of the inner wall (135) in communication with the inner wall (141) through the erythrocyte passageway.

2. The multi-component material separator of Claim 1 wherein the depth filter (138) extends at least from the top edge (137) of the inner wall (135) to the bottom surface (145) of the upper plate (10);
wherein the upper plate (10) has a bottom surface (145), and the depth filter (138) extends to the bottom surface (145).

3. The multi-component material separator of Claim 1 for producing platelet rich plasma concentrate for wound healing, wherein the depth filter (138) has the capacity to accept at least about 85 percent of the hematocrit value of a blood sample and to accept less than a major portion of the platelet rich plasma remaining after the erythrocyte separation from the blood sample.

4. The multi-component material separator of Claim 1 mounted for rotation about an outlet tube (74) concentric with the central axis.

5. The multi-component material separator of Claim 1 wherein the separation chamber (8) includes a balanced array of separator plates (130) extending radially inward from the inner wall and upward from the bottom plate (116), the separation chamber being balanced for substantially vibration-free rotation about the central axis.

6. A multi-component material separator-concentrator comprising the multi-component material separation assembly of Claim 1 in combination with a multi-component material concentrator assembly (11), the concentration assembly having:
a multi-component material concentration sump (162);
an axially concentric rigid stationary outlet tube (74) secured to the housing and extending through the multi-component material separation assembly (3) to the multi-component material concentrate sump (162); and
the multi-component material separation assembly (3) being attached to and positioned above the multi-component material concentration assembly (11) to form a combined separator-concentrator assemblage that is rotatable about the outlet tube (74).

7. The multi-component material separator-concentrator of Claim 6 wherein the PRP concentrator comprises a concentration chamber (18) having a floor (182) for supporting desiccating beads (19) and a wall (180) with at least one opening (186) closed with a screen (188), the screen (188) having openings that are sized to retain the desiccating beads (19) in the concentration chamber (18), the concentration chamber (18) being surrounded by an outer wall (158) with a sloped floor (160) secured thereto, the sloped floor (160) including at its center, a multi-component material concentrate sump (162).

8. The multi-component material separator-concentrator of Claim 6 wherein a stationary bead rake (190) is secured to the stationary tube (74) and extends outward therefrom, the rake having distal ends (198) that are spaced at a distance from the upright screen (188) supports.

9. A process for separating platelet rich plasma from blood sample with a system comprising an inner wall (135) having a top edge (137) and a central axis surrounded by a depth filter (138) having a capacity to receive all of the erythrocytes in the blood sample but insufficient to receive all of the plasma in the blood sample, the inner surface (141) of the inner wall (135) having an angle of at least about 0.2 degrees from the central axis of the inner wall, the method comprising the steps of:
a) spinning an inner cylinder (8) about its central axis at a speed that centrifugally separates erythrocytes from plasma and platelets of the blood sample and causes the erythrocytes to collect against an inner surface of the depth filter (138);
b) continuing spinning for a time sufficient to allow substantially all of the erythrocytes to flow up the inner surface (141) and over the top edge (137) into the depth filter (138), leaving platelet enriched plasma (PRP) behind in the inner cylinder (8); and
c) slowing or discontinuing the spinning to permit the platelet enriched plasma to flow to the bottom of the inner cylinder (8).

10. The process of Claim 9, further comprising:
segmenting the inner surface (141) of the inner wall (135) by radially extending plates (130) to form separation zones (132):
balancing distribution of the blood in the separation zones (132) with the plates (130) during rotation of the separation chamber (8), thereby reducing vibration and erythrocyte displacement from the depth filter (138).

11. The process of Claim 9 wherein the rotational speed of the separation chamber (8) is accelerated to centrifugal speeds at a rate that allows balanced distribution of blood in the separation zones (132), and after the centrifuging is complete, the rotation speed of the separation chamber (8) is decelerated to below centrifugal speeds at a rate that allows balanced distribution of the multi-component material in the separation zones (132), thereby reducing vibration and erythrocyte displacement from the depth filter (138).

12. The process of Claim 9 including the additional step of concentrating the platelet rich plasma by contacting the platelet rich plasma with desiccating beads (19) in a rotating concentration chamber (18) while the beads (19) are agitated with a rake (190) to form a platelet rich plasma concentrate;
wherein the concentration chamber (18) includes an outer screened cylinder (18) confining the desiccating beads (19), and the platelet rich plasma concentrate is separated from the beads (19) by rotating the concentrating chamber (18) about its central axis at a speed that separates platelet rich plasma concentrate from the beads (19).

13. The process of Claim 9 for preparing platelet rich plasma concentrate for wound healing wherein at least about 85 volume percent of the hematocrit value of the blood and less than a major portion of the erythrocyte free platelet rich plasma remaining after the erythrocyte separation is retained by the depth filter (138).

## Patentansprüche

1. Mehrkomponenten-Material-Trenner-Anordnung, die Folgendes beinhaltet:
eine zylindrische, äußere Wand (112), die an der Oberseite durch eine obere Platte (10) geschlossen ist, die eine untere Fläche (145) aufweist, und an der Unterseite geschlossen ist, wobei die äußere Wand eine innere Fläche (136) aufweist;
eine zylindrische, innere Wand (135), die mit der zylindrischen, äußeren Wand (112) konzentrisch ist und eine Oberkante (137) und eine Unterseite aufweist, wobei die innere Wand (135) eine zentrale Achse aufweist, wobei die Unterseite der inneren Wand durch eine geneigte, untere Platte (116) geschlossen ist, die eine zentrale Öffnung (118) aufweist, wobei die untere Platte (116) eine obere Fläche aufweist, die nach unten zu einer zentralen Öffnung (118) geneigt ist, wobei die Oberkante (137) der inneren Wand (135) in einem Abstand von der oberen Platte (145) endet, um einen ringförmigen Erythrozytdurchgang dazwischen zu definieren, wobei die innere Wand (135) eine äußere Fläche (143) aufweist und eine innere Fläche (141) aufweist, die radial von ihrer Oberkante (137) zu ihrer Unterseite in einem Winkel von etwa 0,2 Grad relativ zur zentralen Achse der inneren Wand (135) nach innen geneigt ist; und
einen zylindrischen Tiefenfilter (138), der zwischen der inneren Fläche (136) der äußeren Wand (112) und der äußeren Fläche (143) der inneren Wand (135) in Kommunikation mit der inneren Wand (141) durch den Erythrozytdurchgang positioniert ist.

2. Mehrkomponenten-Material-Trenner gemäß Anspruch 1, wobei sich der Tiefenfilter (138) mindestens von der Oberkante (137) der inneren Wand (135) zur unteren Fläche (145) der oberen Platte (10) erstreckt;
wobei die obere Platte (10) eine untere Fläche (145) aufweist und sich der Tiefenfilter (138) zur unteren Fläche (145) erstreckt.

3. Mehrkomponenten-Material-Trenner gemäß Anspruch 1 zum Produzieren von plättchenreichem Plasmakonzentrat zur Wundheilung, wobei der Tiefenfilter (138) die Kapazität aufweist, um mindestens 85 Prozent des Hämatokritwertes einer Blutprobe zu akzeptieren und weniger als einen Großteil des nach der Erythrozyttrennung von der Blutprobe verbleibenden plättchenreichen Plasmas zu akzeptieren.

4. Mehrkomponenten-Material-Trenner gemäß Anspruch 1, der zur Rotation um ein Auslassrohr (74) konzentrisch mit der zentralen Achse montiert ist.

5. Mehrkomponenten-Material-Trenner gemäß Anspruch 1, wobei die Trennkammer (8) eine balancierte Gruppierung von Trennplatten (130) aufweist, die sich radial von der inneren Wand nach innen erstrecken und von der unteren Platte (116) nach oben erstrecken, wobei die Trennkammer zur im Wesentlichen vibrationsfreien Rotation um die zentrale Achse balanciert ist.

6. Mehrkomponenten-Material-Trenner-Konzentrator, der die Mehrkomponenten-Material-Trennanordnung gemäß Anspruch 1 in Kombination mit einer Mehrkomponenten-Material-Konzentrator-Anordnung (11) beinhaltet, wobei die Konzentrationsanordnung Folgendes aufweist:
eine Mehrkomponenten-Material-Konzentrationswanne (162);
ein axial konzentrisches, starres, stationäres Auslassrohr (74), das am Gehäuse gesichert ist und sich durch die Mehrkomponenten-Material-Trennanordnung (3) zur Mehrkomponenten-Material-Konzentrationswanne (162) erstreckt; und
wobei die Mehrkomponenten-Material-Trennanordnung (3) an der Mehrkomponenten-Material-Konzentrationsanordnung (11) angebracht und über dieser positioniert ist, um eine kombinierte Trenner-Konzentrator-Anordnung zu bilden, die um das Auslassrohr (74) rotierbar ist.

7. Mehrkomponenten-Material-Trenner-Konzentrator gemäß Anspruch 6, wobei der PRP-Konzentrator eine Konzentrationskammer (18) beinhaltet, die einen Boden (182) zum Tragen von austrocknenden Kügelchen (19) und eine Wand (180) mit mindestens einer Öffnung (186), die mit einer Abschirmung (188) geschlossen ist, aufweist, wobei die Abschirmung (188) Öffnungen aufweist, die bemessen sind, um die austrocknenden Kügelchen (19) in der Konzentrationskammer (18) zu halten, wobei die Konzentrationskammer (18) durch eine äußere Wand (158) mit einem daran gesicherten, geneigten Boden (160) umgeben ist, wobei der geneigte Boden (160) in seiner Mitte eine Mehrkomponenten-Material-Konzentrat-Wanne (162) umfasst.

8. Mehrkomponenten-Material-Trenner-Konzentrator gemäß Anspruch 6, wobei ein stationärer Kügelchenrechen (190) am stationären Rohr (74) gesichert ist und sich von diesem nach außen erstreckt, wobei der Rechen (198) distale Enden aufweist, die in einem Abstand von den aufrechten Abstützungen der Abschirmung (188) beabstandet sind.

9. Prozess zum Trennen von plättchenreichem Plasma von einer Blutprobe mit einem System, beinhaltend eine innere Wand (135), die eine Oberkante (137) und eine zentrale Achse aufweist, umgeben von einem Tiefenfilter (138), der eine Kapazität aufweist, um alle Erythrozyten in der Blutprobe aufzunehmen, aber unzureichend, um das ganze Plasma der Blutprobe aufzunehmen, wobei die innere Fläche (141) der inneren Wand (135) einen Winkel von mindestens etwa 0,2 Grad von der zentralen Achse der inneren Wand aufweist, wobei das Verfahren die folgenden Schritte beinhaltet:
a) Spinnen eines inneren Zylinders (8) um seine zentrale Achse mit einer Geschwindigkeit, die zentrifugal Erythrozyten von Plasma und Plättchen der Blutprobe trennt und verursacht, dass sich die Erythrozyten an einer inneren Fläche des Tiefenfilters (138) sammeln;
b) Fortsetzen des Spinnens für eine Zeit, die ausreicht, um zu erlauben, dass im Wesentlichen alle Erythrozyten die innere Fläche (141) hinauf und über die Oberkante (137) in den Tiefenfilter (138) hinein strömen, wodurch plättchenangereichertes Plasma (Platelet Enriched Plasma, PRP) im inneren Zylinder (8) zurückbleibt; und
c) Verlangsamen oder Unterbrechen des Spinnens, um zu gestatten, dass das plättchenangereicherte Plasma zur Unterseite des inneren Zylinders (8) strömt.

10. Prozess gemäß Anspruch 9, das ferner Folgendes beinhaltet:
Segmentieren der inneren Fläche (141) der inneren Wand (135) durch sich radial erstreckende Platten (130), um Trennzonen (132) zu bilden;
Balancieren der Verteilung des Blutes in den Trennzonen (132) mit den Platten (130) während der Rotation der Trennkammer (8), wodurch Vibration und Erythrocytverdrängung vom Tiefenfilter (138) reduziert wird.

11. Prozess gemäß Anspruch 9, wobei die Rotationsgeschwindigkeit der Trennkammer (8) auf zentrifugale Geschwindigkeiten beschleunigt wird, mit einer Rate, die balancierte Verteilung von Blut in den Trennzonen (132) erlaubt, und nachdem das Zentrifugieren abgeschlossen ist, die Rotationsgeschwindigkeit der Trennkammer (8) auf unter die zentrifugalen Geschwindigkeiten entschleunigt wird, mit einer Rate, die Verteilung des Mehrkomponenten-Materials in den Trennzonen (132) erlaubt, wodurch Vibration und Erythrocytverdrängung vom Tiefenfilter (138) reduziert wird.

12. Prozess gemäß Anspruch 9, der den zusätzlichen Schritt des Konzentrierens des plättchenreichen Plasmas durch Inkontaktbringen des plättchenreichen Plasmas mit austrocknenden Kügelchen (19) in einer rotierenden Konzentrationskammer (18), während die Kügelchen (19) mit einem Rechen (190) gerührt werden, umfasst, um ein plättchenreiches Plasmakonzentrat zu bilden;
wobei die Konzentrationskammer (18) einen äußeren abgeschirmten Zylinder (18) umfasst, der die austrocknenden Kügelchen (19) einschließt, und das plättchenreiche Plasmakonzentrat von den Kügelchen (19) durch Rotieren der Konzentrierkammer (18) um ihre zentrale Achse mit einer Geschwindigkeit getrennt wird, die plättchenreiches Plasmakonzentrat von den Kügelchen (19) trennt.

13. Verfahren gemäß Anspruch 9 zum Aufbereiten von plättchenreichem Plasmakonzentrat zur Wundheilung, wobei mindestens 85 Volumenprozent des Hämatokritwertes des Blutes und weniger als ein Großteil des nach der Erythrozyttrennung verbleibenden erythrocytfreien plättchenreichen Plasmas durch den Tiefenfilter (138) gehalten werden.

## Revendications

1. Ensemble séparateur de substance à plusieurs composants comprenant :
une paroi externe cylindrique (112) fermée au niveau supérieur par une plaque supérieure (10) comportant une surface inférieure (145) et fermée au niveau inférieur, la paroi externe comportant une surface interne (136) ;
une paroi interne cylindrique (135) concentrique par rapport à la paroi externe cylindrique (112) et comportant un bord supérieur (137) et une partie inférieure, la paroi interne (135) possédant un axe central, la partie inférieure de la paroi interne étant fermée par une plaque inférieure inclinée (116) comportant une ouverture centrale (118), la plaque inférieure (116) comportant une surface supérieure inclinée en direction d'une ouverture centrale (118), le bord supérieur (137) de la paroi interne (135) se terminant à une certaine distance de la plaque supérieure (145) pour définir un passage annulaire pour érythrocytes entre ces derniers, la paroi interne (135) comportant une surface externe (143) et comportant une surface interne (141) qui est inclinée radialement vers l'intérieur depuis son bord supérieur (137) jusqu'à sa partie inférieur suivant un angle d'environ 0,2 degré par rapport à l'axe central de la paroi interne (135) ; et
un filtre en profondeur cylindrique (138) placé entre la surface interne (136) de la paroi externe (112) et la surface externe (143) de la paroi interne (135) en communication avec la paroi interne (141) à travers le passage pour érythrocytes.

2. Séparateur de substance à plusieurs composants selon la revendication 1, ledit filtre en profondeur (138) s'étendant au moins du bord supérieur (137) de la paroi interne (135) jusqu'à la surface inférieure (145) de la plaque supérieure (10) ; ladite plaque supérieure (10) comportant une surface inférieure (145), et ledit filtre en profondeur (138) s'étendant jusqu'à la surface inférieure (145).

3. Séparateur de substance à plusieurs composants selon la revendication 1 destiné à la production d'un concentré de plasma riche en plaquettes pour la cicatrisation des plaies, ledit filtre en profondeur (138) ayant la capacité d'accepter au moins environ 85 pour cent de la valeur d'hématocrite d'un échantillon de sang et d'accepter moins de la majeure partie du plasma riche en plaquettes restant après la séparation des érythrocytes de l'échantillon de sang.

4. Séparateur de substance à plusieurs composants selon la revendication 1 monté pour la rotation autour d'un tube de sortie (74) concentrique avec l'axe central.

5. Séparateur de substance à plusieurs composants selon la revendication 1, la chambre de séparation (8) comprenant une série équilibrée de plaques séparatrices (130) s'étendant radialement vers l'intérieur depuis la paroi interne et vers le haut depuis la plaque inférieure (116), la chambre de séparation étant équilibrée pour une rotation pratiquement exempte de vibrations autour de l'axe central.

6. Séparateur-concentrateur de substance à plusieurs composants comprenant l'ensemble séparateur de substance à plusieurs composants selon la revendication 1 en association avec un ensemble concentrateur de substance à plusieurs composants (11), l'ensemble de concentration comportant :
un puisard de concentration de substance à plusieurs composants (162) ;
un tube de sortie fixe rigide axialement concentrique (74) fixé au logement et s'étendant à travers l'ensemble de séparation de substance à plusieurs composants (3) jusqu'au puisard de concentration de substance à plusieurs composants (162) ; et
l'ensemble de séparation de substance à plusieurs composants (3) étant fixé et positionné au-dessus de l'ensemble de concentration de substance à plusieurs composants (11) pour former un assemblage séparateur-concentrateur combiné qui peut tourner autour du tube de sortie (74).

7. Séparateur-concentrateur de substance à plusieurs composants selon la revendication 6, le concentrateur de PRP comprenant une chambre de concentration (18) comportant un plancher (182) destiné à supporter des billes déshydratantes (19) et une paroi (180) dotée d'au moins une ouverture (186) fermée par un tamis (188), le tamis (188) comportant des ouvertures qui sont dimensionnées pour retenir les billes déshydratantes (19) dans la chambre de concentration (18), la chambre de concentration (18) étant entourée d'une paroi externe (158) à laquelle est fixé un plancher incliné (160), le plancher incliné (160) comprenant en son centre un puisard de concentré de substance à plusieurs composants (162).

8. Séparateur-concentrateur de substance à plusieurs composants selon la revendication 6, un agitateur à billes fixe (190) étant fixé au tube fixe (74) et s'étendant vers l'extérieur depuis celui-ci, l'agitateur possédant des extrémités distales (198) qui sont espacées à une certaine distance des supports verticaux du tamis (188).

9. Procédé de séparation de plasma riche en plaquettes d'un échantillon de sang avec un système comprenant une paroi interne (135) comportant un bord supérieur (137) et un axe central entouré d'un filtre en profondeur (138) ayant une capacité à recevoir tous les érythrocytes présents dans l'échantillon de sang mais insuffisant pour recevoir tout le plasma présent dans l'échantillon de sang, la surface interne (141) de la paroi interne (135) faisant un angle d'au moins environ 0,2 degré avec l'axe central de la paroi interne, le procédé comprenant les étapes de :
a) rotation d'un cylindre interne (8) autour de son axe central à une vitesse qui sépare par centrifugation les érythrocytes du plasma et les plaquettes de l'échantillon sanguin et entraîne la collecte des érythrocytes contre une surface interne du filtre en profondeur (138) ;
b) poursuite de la rotation pendant une durée suffisante pour permettre à pratiquement tous les érythrocytes de remonter la surface interne (141) et au-dessus du bord supérieur (137) dans le filtre en profondeur (138), laissant le plasma enrichi en plaquettes (PRP) derrière le cylindre interne (8) ; et
c) poursuite ou interruption de la rotation pour permettre au plasma enrichi en plaquettes de s'écouler jusqu'à la partie inférieure du cylindre interne (8).

10. Procédé selon la revendication 9, comprenant en outre :
la segmentation de la surface interne (141) de la paroi interne (135) par des plaques s'étendant radialement (130) pour former des zones de séparation (132) ;
l'équilibrage de la distribution du sang dans les zones de séparation (132) avec les plaques (130) au cours de la rotation de la chambre de séparation (8), réduisant ainsi les vibrations et le déplacement des érythrocytes depuis le filtre en profondeur (138).

11. Procédé selon la revendication 9, la vitesse de rotation de la chambre de séparation (8) étant accélérée jusqu'à des vitesses centrifuges à un taux qui permet une distribution équilibrée du sang dans les zones de séparation (132), et une fois la centrifugation terminée, la vitesse de rotation de la chambre de séparation (8) est décélérée en-dessous de vitesses centrifuges à un taux qui permet une distribution équilibrée de la substance à plusieurs composants dans les zones de séparation (132), réduisant ainsi les vibrations et le déplacement des érythrocytes depuis le filtre en profondeur (138).

12. Procédé selon la revendication 9 comprenant l'étape supplémentaire de concentration du plasma riche en plaquettes par la mise en contact du plasma riche en plaquettes avec des billes déshydratantes (19) dans une chambre de concentration rotative (18) alors que les billes (19) sont agitées avec un agitateur (190) pour former un concentré de plasma riche en plaquettes ;
ladite chambre de concentration (18) comportant un cylindre criblé externe (18) confinant les billes déshydratantes (19), et ledit concentré de plasma riche en plaquettes étant séparé des billes (19) par rotation de chambre de concentration (18) autour de son axe central à une vitesse qui sépare le concentré de plasma riche en plaquettes des billes (19).

13. Procédé selon la revendication 9 permettant la préparation d'un concentre de plasma riche en plaquettes pour la cicatrisation des plaies, au moins environ 85 pour cent en volume de la valeur d'hématocrite du sang et moins de la majeure partie du plasma riche en plaquettes exempt d'érythrocytes restant après la séparation des érythrocytes étant retenue par le filtre en profondeur (138).
